# EUROPEAN PATENT APPLICATION

(11) **EP 4 781 945 A1**
(43) Date of publication of application: **29.07.2026**
(21) Application number: 25207898.5
(22) Date of filing: 10.10.2025
(51) Int. Cl.: A61C 9/00, A61C 8/00

(54) **A DENTAL SCAN AID**

(30) Priority: 22.01.2025 GB 202500913
(71) Applicant: Nulty, Adam Brian, Hethel, Norwich NR14 8FB (GB)
(72) Inventor: Nulty, Adam Brian, Hethel, Norwich NR14 8FB (GB)

(57) **Abstract**

A dental scan aid 12 is provided comprising a scan substrate 14 having a randomised irregular surface 16 unique to the dental scan aid 12. There is a connection means to allow the scan substrate 14 to be fixed relative to a patient's maxillary or mandibular arch 10a, 10b. There is also a regular structure 24 extending from the randomised irregular surface 16 to thereby provide fixed positional information relative to the maxillary or mandibular arch 10a, 10b to improve dental scan accuracy when the dental scan aid 12 is in position.

## Description

The present invention relates to a dental scan aid for enhancing scanning of a patient's jaw. In particular, this invention relates to intraoral scanning aids used in dental procedures, addressing challenges in scanner recognition, stability, reusability, and digital workflow efficiency for full-arch and partial-arch scanning on multi-unit abutments.

Existing intraoral scanning aids often encounter the following issues:
1. Scanner Confusion: Identical scan aids on multiple abutments can confuse scanners, leading to misaligned data stitching.
2. Rotational Instability: Long cantilever scan aids rotate under pressure, requiring scan restarts.
3. CAD Workflow Complexity: Varying scan body designs require multiple CAD libraries, increasing complexity and error risks.
4. Durability Issues: Worn screws often necessitate replacement of the entire scan body, increasing costs and waste.

It is an object of the present invention to obviate or overcome the above-referenced difficulties.

According to a first aspect of the invention, there is provided a dental scan aid for enhancing a scan accuracy of a maxillary or mandibular arch during a dental scan procedure, the dental scan aid comprising: a scan substrate has a randomised irregular surface unique to the dental scan aid; connection means to allow the scan substrate to be fixed relative to the said maxillary or mandibular arch; and characterised in that the scan substrate further includes a regular structure extending above the randomised irregular surface to thereby provide a fixed positional information relative to the maxillary or mandibular arch to improve dental scan accuracy during a dental scan procedure.

Dental scan aids are known which assist with scanning of a patient's oral region and which then allow for mapping of the maxillary and mandibular arches for subsequent dental work. However, difficulties lie in the correct referencing of the scan data with respect to the dental scan aids. The use of unique randomised irregular surfaces, in conjunction with a fixed regular structure against which the orientation of the randomised irregular surface can be determined, allows for rapid modelling of the maxillary or mandibular arch in CAD software, allowing for accurate generation of subsequent dental prosthetics.

Optionally, the regular structure may be provided as a cylinder.

A cylinder is an easily registrable structure which provides a convenient anchor point for a fastener and can be readily recognised by image recognition software which enables the construction of a 3D model of the patient's maxillary or mandibular arch using CAD software or artificial intelligence.

In one preferable embodiment the connection means may be co-located with the regular structure.

The positioning of the regular structure is designed to be the fixed location of the dental scan aid. This allows the CAD software to rapidly determine orientation of the dental scan aid based on the regular structure.

Optionally, the regular structure may be located at a geometric centre of the scan substrate.

Positioning of the regular structure in the centre of the dental scan aid may improve the speed and accuracy of the assignment of the orientation in the CAD software.

Preferably the regular structure may comprise a fiducial marker.

The regular structure may have a colour contrast to the randomised irregular surface to improve scan visibility.

There are several mechanisms by which scan fidelity can be improved, which can be integrated as part of the

Optionally, the regular structure may include one or more reinforcement members.

The torque transmitted through the screw-threaded fastener may cause deformation to the regular structure.

The regular structure may be releasably engagable with the scan substrate.

Modularisation of the dental scan aid may allow for different randomised portions to be engaged with the same regular structure in an effort to more rapidly and accurately scan a particular region within the patient's mouth.

The regular structure may include reflective material for improved scanner recognition.

Reflectivity for the scanner wavelength may be a desirable property for the regular structure in particular, considering this is the reference component of the dental scan aid.

The randomised irregular surface may comprise a randomised topography having minima and maxima of different heights relative to the scan substrate.

A topographically varied randomised irregular surface has the advantage of the Preferably, the randomised irregular surface may comprise at least one channel extending to an edge of the scan substrate for encouraging saliva run-off.

Since saliva pooling in the dental scan aid can reduce the effectiveness of the scanning process, it is desirable to allow some form of run-off means to remove saliva during the dental process.

In one embodiment, the connection means may comprise a screw-threaded fastener and a fastener receiver through the scan substrate.

The fastener holds the dental scan aid in a fixed position relative to the maxillary or mandibular arch for accurate scanning.

Optionally, the screw-threaded fastener may comprise a bevelled shank and the fastener receiver may then have a complementarily-shaped receiver.

The bevel of the shank allows for a force to be more readily transferred to the dental scan aid, holding the dental scan aid more securely thus preventing movement in the mouth during a scan.

Preferably, the screw-threaded fastener may have a self-locking thread to reduce a risk of loosening during use.

It is desirable to ensure that the fasteners do not disengage during use, and therefore a self-locking or tapping thread can reduce this risk, minimising scan artefacts.

Optionally, the screw-threaded fastener may have a toolless engagement means.

This may allow for a more straightforward connection mechanism.

The scan substrate may comprise an abutment seat configured to engage with an abutment element fixed into a patient's maxillary or mandibular arch.

Most dental procedures of this type will use abutment elements, sometimes referred to as multi unit abutments, and therefore interfacing between the dental scan aids and the abutments will simplify use.

Optionally, the abutment seat may comprise an anti-rotation element.

Anti-rotation fins or similar may reduce the risk of movement of the dental scan aid during a scan process.

In one arrangement, the abutment seat may be releasably engagable with the scan substrate.

Modularisation of the dental scan aid may allow for the randomised irregular surfaces to be swapped for most efficient scanning properties, even during a dental procedure.

Optionally, the abutment seat may be integrally formed with the regular structure.

It may be that the regular structure and the abutment seat are formed together, for structural integrity, reducing the risk of movement relative to one another during use.

The scan substrate may comprise an anti-reflective coating.

Reducing glare or scan artefacts by the use of anti-reflective coating can improve the effectiveness of the scanning process.

The dental scan aid as claimed in any one of the preceding claims, further comprising one or more fins or stabilising elements engaged with the scan substrate.

It is desirable to limit flexing or bending actions on the scan substrate which might create scanning artefacts, and this can be achieved by supporting the scan substrate against the gums of the patient.

A dental scan aid may further comprise an integrated RFID or NFC communications element embedded in the scan substrate.

Wireless communication protocols may allow for additional metadata from the dental scan aid to be obtained, assisting with the post-scan generation of a CAD model.

The randomised irregular surface may comprise one or more notches.

Notches provide additional unique surface structure to improve the identification of the specific dental scan aid during scanning and processing.

According to a second aspect of the invention, there is provided a dental scan aid system comprising a plurality of dental scan aids in accordance with the first aspect of the invention, wherein the randomised irregular surface of each of the dental scan aids is different.

According to a third aspect of the invention, there is provided a method of improving the accuracy of interpretation of scan data of a maxillary or mandibular arch taken during a dental scan procedure, the method comprising the steps of: a] obtaining scan data comprising a plurality of scans of a patient having at least one dental scan aid according to the first aspect of the invention attached to their maxillary or mandibular arch, the plurality of scans each including both the randomised irregular surface and the regular structure of the or each dental scan aid; and b] using the regular structure as an alignment landmark, using computer software to build a 3D model of the maxillary or mandibular arch using the randomised irregular surfaces as a reference.

The invention will now be more particularly described, by way of example only, with reference to the accompanying drawings, in which:
Figure 1A shows a plan view of an exemplary maxillary arch of a patient having a plurality of dental scan aids in accordance with the first aspect of the invention attached thereto;
Figure 1B shows a plan view of an exemplary mandibular arch of a patient having a plurality of dental scan aids in accordance with the first aspect of the invention attached thereto;
Figure 2 shows a plan view of one of the dental scan aids of Figure 1A;
Figure 3 shows a side view of the dental scan aid of Figure 2;
Figure 4 shows a vertical cross-section through a dental scan aid in accordance with the first aspect of the invention; and
Figure 5 shows a plan view of an exemplary mandibular arch of a patient having a plurality of different dental scan aids in accordance with the first aspect of the invention.

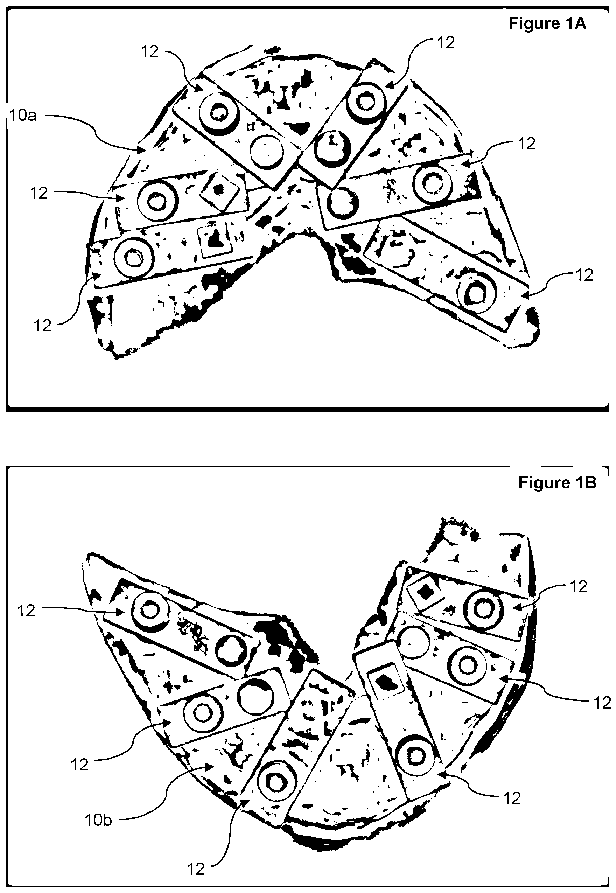

Referring to Figures 1A and 1B, there is indicated examples of maxillary and mandibular arches 10a, 10b of a patient, each having a plurality of dental scan aids 12 associated therewith.

Each dental scan aid 12 has a scan substrate 14 having a randomised irregular surface 16 which is unique, so that the dental scan aid 12 can be readily identified from a library of dental scan aids 12. The randomised irregular surface 16 may extend only on the upper surface of the dental scan aid 12, that is, the surface in-use furthest from the gums of the patient.

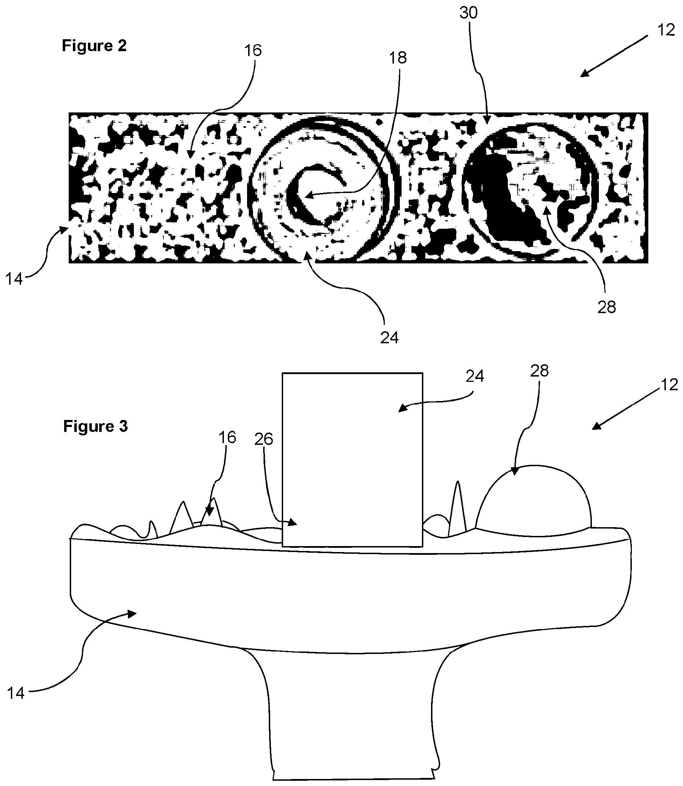

The dental scan aid 12 is shown more clearly in Figures 2 and 3. The dental scan aid 12 comprises a connection means, here in the form of a fastener receiver 18 through the scan substrate 14 for receiving a screw-threaded fastener 20 therethrough. This allows for connection directly into the maxillary and mandibular arches 10a, 10b, or more likely, into an abutment element 22 which acts as the interface between the maxillary and mandibular arches 10a, 10b and the dental scan aid 12.

The scan substrate 14 further comprises a regular structure 24, preferably in the form of a cylinder or generally cylindrical object, which extends from the randomised irregular surface 16. The term regular structure 24 here refers to an easily identifiable geometric three-dimensional object, most preferably having a clearly defined symmetry, particularly rotational symmetry. This is in stark contrast to the randomised irregular surface 16, which will almost certainly be asymmetric in form.

The purpose of the regular structure 24 is to act as an alignment landmark during a scan process, so that the scanning rapidly identifies the position of the dental scan aid 12, with the randomised irregular surface 16 providing an easily identifiable orientation identifier of the dental scan aid 12.

The fastener receiver 18 is positioned so as to be co-located with the regular structure 24. This allows for the anchoring of the dental scan aid 12, and ensures that the position of the regular structure 24 does not move during a scan. This improves the accuracy of the registration process when scan images are stitched to form a full 3D model in due course. It is preferred that this be at the geometric centre of the scan substrate 14, to further simplify the recognition of the dental scan aid 12 in use. In the depicted embodiment, the dental scan aid 12 has a scan substrate 14 which is or is substantially rectilinear, which makes identification of a geometric centre straightforward. However, alternative scan substrate 14 shapes are feasible.

There are various additional features which may be desirable to provide as part of the regular structure 24. For instance, the regular structure 24 may have a colour contrast to the randomised irregular surface 16 to improve scan visibility. Textural or material contrast could also provide such a differentiation. There may be one or more fiducial markers on the surface of the regular structure 24 which are easily identifiable, but which do not affect or substantially affect the shape and therefore recognisability of the regular structure 24. This improves photogrammetry compatibility. There may also be reinforcement members, where applicable, which help to prevent damage to the regular structure 24 as the torque is applied via the screw-threaded fastener 20.

The regular structure 24 may be modular and interchangeable so as to accommodate different arrangements. This may manifest as releasable engagement with the scan substrate 14 itself.

Micro-indentations or perforations in a surface of the regular structure 24 may improve the ability to enhance scanner light reflection and improve scan accuracy. Additionally, or alternatively, such micro-indentations or perforations may be provided on the randomised irregular surface 16.

To further improve the scan characteristics of the 12, it may be desirable to embed the regular structure with reflective material so as to improve the scanner recognition thereof. Another possibility may be to extend the randomised irregular surface 16 onto an external surface 26 of the regular structure 24 so as to provide improved scanner recognition thereof from multiple angles. Additionally, or alternatively, there may be additional markings or indentations on the regular structure 24 for enhanced scanner accuracy during alignment.

The randomised irregular surface 16 may applied to one or more surfaces of the dental scan aid 12, and has variations in degree, that is, having major and minor surface irregularities, akin to a topographical landscape. The scale of the irregularities differs across the system 50 as a whole, with each dental scan aid 12 being unique. The randomised irregular surface 16 can minimise shadowing effects and improve overall scan accuracy.

The randomised irregular surface 16 may include one or more embedded geometric shapes 28, such as the hemisphere shown in Figures 2 and 3, for improved scanner recognition. In particular, asymmetric features of the randomised irregular surface 16 may allow for identification of the dental scan aid 12 during an only partially complete scan. Other features which could achieve similar goals could be the incorporation of symbols or letters for automatic scanner recognition. Indeed, the randomised irregular surface 16 could incorporate specific features to reduce glare or reflective artefacts during a scanning process.

The randomised irregular surface 16 may include one or more grooves or channels 30 thereon, which preferably extend to an edge of the scan substrate 14. This is designed to improve saliva run-off from the randomised irregular surface 16, which may otherwise have a tendency to pool, and thus inhibit scan accuracy.

The surface of the randomised irregular surface 16 may have tactile indicators which assist the dental practitioner with manual alignment in the patient's mouth.

The skilled practitioner may also be able to modify the randomised irregular surface 16, most commonly by drilling, during the course of a dental procedure, to improve the scan capabilities of the dental scan aid 12 chosen.

One or more additional secondary alignment features could be provided, such as a ridge or notch, which provides redundancy during CAD alignment during a rotational adjustment process.

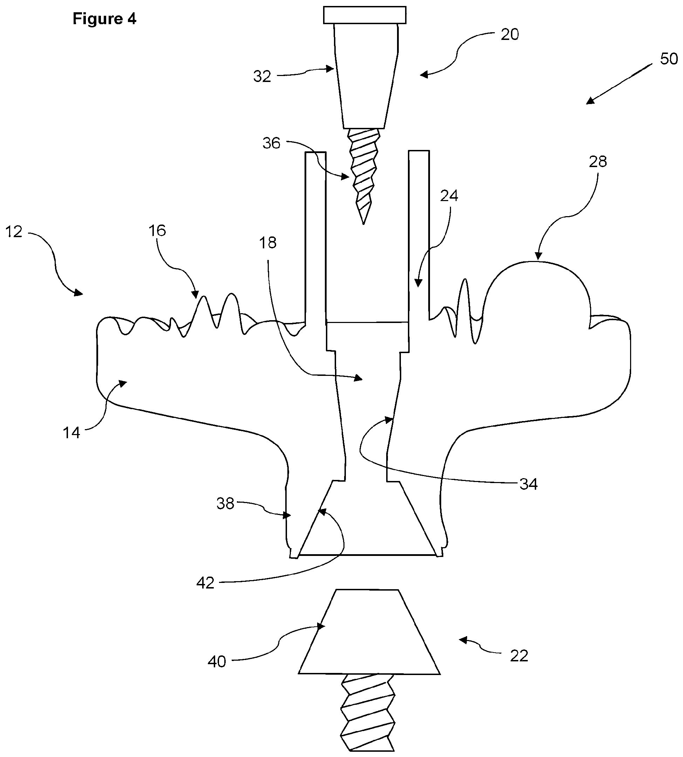

Figure 4 shows the assembly of a dental scan aid 12 in conjunction with the abutment element 22 which mounts to the maxillary or mandibular arch 10a, 10b, and the screw-threaded fastener 20.

The screw-threaded fastener 20 is complementarily-shaped to the counterpart fastener receiver 18 of the scan substrate 14. In this instance, the screw-threaded fastener 20 has a bevelled shank 32, with the fastener receiver 18 having a counterpart bevelled portion 34 which the bevelled shank 32 seats into. This enables a dual-contact locking system of the screw-threaded fastener 20 with both the scan substrate 14 and the abutment element 22 for secure locking.

The screw-threaded fastener 20 may advantageously have a self-locking thread 36 to reduce a risk of loosening during use, and may engage in a toolless manner, for instance, via a snap-fit mechanism with the abutment element 22. The screw-threaded fastener 20 may include torque-limiting features to prevent or reduce overtightening into the abutment element 22 and/or the maxillary or mandibular arch 10a, 10b.

Since the system 50 is constructed in a modular fashion, the screw-threaded fastener 20 can be removed, sanitised, and re-used with different components.

The screw-threaded fastener 20 is formed from or coated with a wear-resistant material, thereby allowing autoclaving thereof when sanitisation is required. The screw-threaded fastener 20 should be sufficiently robust to withstand multiple autoclave cycles.

The abutment seat 38 is where the abutment element 22 engages with the dental scan aid 12, and is formed so as to depend from the scan substrate 14. The abutment element 22 may have a tapered head 40, and/or may have one or more anti-rotation elements thereon, which engages with a complementary receiver 42 of the abutment seat 38. The complementary receiver 42 may have grooves or channels therein which mate with the abutment element 22 to assist with alignment.

The abutment seat 38 may be formed so as to be releasably engagable with the scan substrate 14. The abutment seat 38 could, for instance, be integrally formed with the regular structure 24.

The abutment seat 38 and/or the scan substrate 14 may be provided with additional stabilising members, such as fins or extensions which aim to limit or prevent lateral movement of the 12 during a procedure.

The abutment seat 38 is designed to be ergonomic so as to cause the least amount of patient discomfort when engaged with an abutment element 22.

Using the dental scan aids 12 as herebefore described, there is a method of improving the accuracy of interpretation of scan data of a maxillary or mandibular arch 10a, 10b taken during a dental scan procedure. The method comprises the steps of obtaining scan data comprising a plurality of scans of a patient having at least one dental scan aid 12 attached to their maxillary or mandibular arch 10a, 10b, the plurality of scans each including both the randomised irregular surface 16 and the regular structure 24 of the or each dental scan aid 12. The regular structure 24 can then be used as an alignment landmark, using computer software to build a 3D model of the maxillary or mandibular arch 10a, 10b using the randomised irregular surfaces 16 as a reference.

A system comprising a plurality of dental scan aids 12, each having a unique randomised irregular surface 16 thus allows for stitching together of images to form a representation of the patient's maxillary or mandibular arch 10a, 10b.

Each of the plurality of dental scan aids 12 has a common central structure, which is preferably in the form of a cylinder.

The regular structure 24 extends above the level of the randomised irregular surface 16.

Specifically, the apex of the cylinder importantly and crucially projects above the highest point of the surrounding irregular surface 16 which provides a good amount of material to have a common reference cylinder portion. The cylinder's dimensional tolerances (height, diameter, concentricity) are constant across an entire family of scan bodies 10

The irregular surface of the scan substrate 14 provides for uniqueness and ease of identification of a specific scan aid 12 within the system, and a common geometry of a cylindrical regular structure 24 which extends up and away from the irregular surface 14 to allow for improved CAD library matching. The present invention introduces a unifying cylindrical reference 24 within each irregular body 12, meaning CAD software only needs to recognise the common shape, solving both cross-arch stitching issues and the library complexity problem.

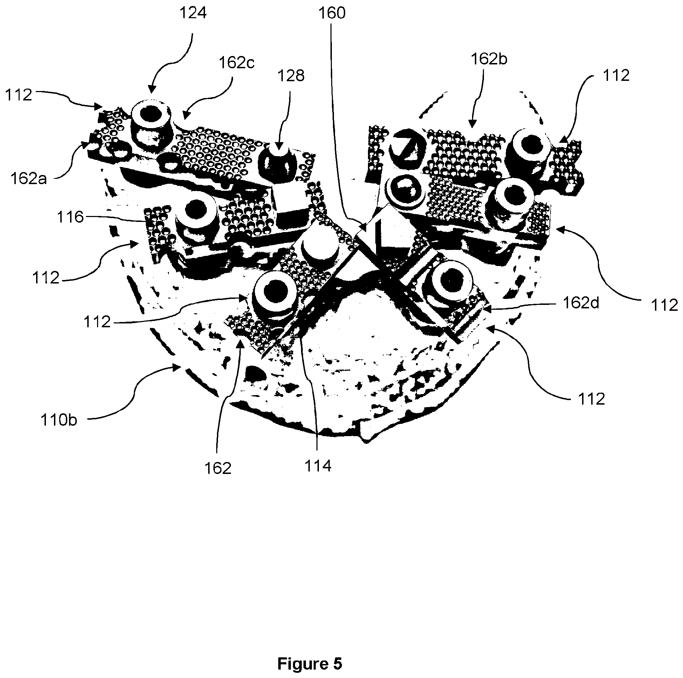

A second embodiment of dental scan aids 112 are indicated in a mandibular arch 110b in Figure 5. Identical or similar components will be referenced using identical or similar reference numerals, and further detailed description is omitted for brevity.

Each dental scan aid 112 includes a substantially rectilinear scan substrate 114 and a cylindrical regular structure 124 which extends upwards above the entire top surface of the scan substrate 114. The randomised irregular surface 116 is provided on the top surface of the scan substrate 114.

The randomised irregular surface 116 is here formed as a series of indentations in the scan substrate 114 in a regular patten, but having an irregular and/or random coverage of the area of the top surface of the scan substrate 114. This provides the improved unique identifiability of the dental scan aid 112 within the set.

Each dental scan aid 112 has a different embedded geometric shape 128, and may typically be at least partially contacting a non-indented portion 160 of the randomised irregular surface 116.

Additionally, the randomised irregular surface 116 may be made more unique by the inclusion of one or more notches 162 or recesses. This may include notches 162a which extend partway of a depth into the scan substrate 114, notches 162b which extend through an entire depth of the scan substrate 114, notches 162c which extend part of the way into a width of the scan substrate 114, and notches 162d which span the entire width of the scan substrate 114.

The irregularity of the notches 162 provide an improved means of uniquely identifying which dental scan aid 112 has been scanned during a scanning procedure. This avoids any issues with any repeated geometric features otherwise on the dental scan aid 112 from confusing the scanning processor.

Any or all of the components of the system may be provided with anti-reflective coatings designed to reduce glare and artefacts during a scanning process.

The dental scan aid may be provided with integrated radio frequency (RFID) or near field communication (NFC) chips embedded within the scan substrate, and in particular in the regular structure, which can then provide additional metadata for CAD alignment when preparing a 3D model.

**It** is therefore possible to provide a means of improving the accuracy with which a dental scanning procedure can be performed, particularly for a maxillary or mandibular arch, by the use of dental scan aids which have both a regular portion which can act as a landmark, and a randomised irregular surface which is uniquely identifiable.

The words 'comprises/comprising' and the words 'having/including' when used herein with reference to the present invention are used to specify the presence of stated features, integers, steps, or components, but do not preclude the presence or addition of one or more other features, integers, steps, components, or groups thereof.

**It** is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of examples only, and various other modifications will be apparent to persons skilled in the field without departing from the scope of the invention as defined herein.

## Claims

1. A dental scan aid (12; 112) for enhancing a scan accuracy of a maxillary or mandibular arch (10a, 10b; 110b) during a dental scan procedure, the dental scan aid (12; 112) comprising:
a scan substrate (14; 114) has a randomised irregular surface (16; 116) unique to the dental scan aid (12; 112);
connection means to allow the scan substrate (14; 114) to be fixed relative to the said maxillary or mandibular arch (10a, 10b; 110b); and
**characterised in that**
the scan substrate (14; 114) further includes a regular structure (24; 124) extending above the randomised irregular surface (16; 116) to thereby provide a fixed positional information relative to the maxillary or mandibular arch (10a, 10b; 110b) to improve dental scan accuracy during a dental scan procedure.

2. A dental scan aid (12; 112) as claimed in claim 1, wherein the regular structure (24; 124) is provided as a cylinder.

3. A dental scan aid (12; 112) as claimed in claim 1 or claim 2, wherein the connection means is co-located with the regular structure (24; 124).

4. A dental scan aid (12; 112) as claimed in any one of the preceding claims, wherein the regular structure (24; 124) is located at a geometric centre of the scan substrate (14; 114).

5. A dental scan aid (12; 112) as claimed in any one of the preceding claims, wherein the regular structure (24; 124) comprises a fiducial marker.

6. A dental scan aid (12; 112) as claimed in any one of the preceding claims, wherein the regular structure (24; 124) has a colour contrast to the randomised irregular surface (16; 116) to improve scan visibility.

7. A dental scan aid (12; 112) as claimed in any one of the preceding claims, wherein the regular structure (24; 124) is releasably engagable with the scan substrate.

8. A dental scan aid (12; 112) as claimed in any one of the preceding claims, wherein the regular structure (24; 124) includes reflective material for improved scanner recognition.

9. A dental scan aid (12; 112) as claimed in any one of the preceding claims, wherein the randomised irregular surface (16; 116) comprises a randomised topography having minima and maxima of different heights relative to the scan substrate (14; 114).

10. A dental scan aid (12; 112) as claimed in any one of the preceding claims, wherein the connection means comprises a screw-threaded fastener (20) and a fastener receiver (18) through the scan substrate (14; 114).

11. A dental scan aid (12; 112) as claimed in any one of the preceding claims, wherein the scan substrate (14; 114) comprises an abutment seat (38) configured to engage with an abutment element (22) fixed into a patient's maxillary or mandibular arch (10a, 10b; 110b).

12. A dental scan aid (12; 112) as claimed in any one of the preceding claims, wherein the scan substrate (14; 114) comprises an anti-reflective coating.

13. A dental scan aid (12; 112) as claimed in any one of the preceding claims, wherein the randomised irregular surface (16; 116) comprises one or more notches (162).

14. A dental scan aid system comprising a plurality of dental scan aids (12; 112) as claimed in any one of the preceding claims, wherein the randomised irregular surface (16; 116) of each of the dental scan aids (12; 112) is different.

15. A method of improving the accuracy of interpretation of scan data of a maxillary or mandibular arch taken during a dental scan procedure, the method comprising the steps of:
a] obtaining scan data comprising a plurality of scans of a patient having at least one dental scan aid (12; 112) according to any one of claims 1 to 13 attached to their maxillary or mandibular arch (10a, 10b; 110b), the plurality of scans each including both the randomised irregular surface (16; 116) and the regular structure (24; 124) of the or each dental scan aid (12; 112); and
b] using the regular structure (24; 124) as an alignment landmark, using computer software to build a 3D model of the maxillary or mandibular arch (10a, 10b; 110b) using the randomised irregular surfaces (16; 116) as a reference.
